**Europäisches Patentamt**

**European Patent Office**

**Office européen des brevets**

(11) Veröffentlichungsnummer: **0 101 534**

**B1**

(12) **EUROPÄISCHE PATENTSCHRIFT**

(45) Veröffentlichungstag der Patentschrift:
**13.08.86**

(21) Anmeldenummer: **83105273.3**

(22) Anmeldetag: **27.05.83**

(51) Int. Cl.⁴: **A 61 M 5/31,** A 61 M 25/00

(54) **Ventilvorrichtung.**

(30) Priorität: **31.07.82 DE 8223689 U**

(43) Veröffentlichungstag der Anmeldung:
**29.02.84 Patentblatt 84/9**

(45) Bekanntmachung des Hinweises auf die Patenterteilung:
**13.08.86 Patentblatt 86/33**

(84) Benannte Vertragsstaaten:
**AT BE CH DE FR GB IT LI LU NL SE**

(56) Entgegenhaltungen:
**DE-A-2 514 929**
**DE-A-2 623 511**
**DE-A-2 750 454**
**US-A-3 357 674**
**US-A-4 215 702**
**US-A-4 333 479**

(73) Patentinhaber: **INTERMEDICAT GMBH,**
**Gerliswilstrasse 74, CH- 6020 Emmenbrücke (CH)**

(72) Erfinder: **Brencher, Norbert, Brandenburgerstrasse**
**22, D-3436 Hess. Lichtenau (DE)**

(74) Vertreter: **von Kreisler, Alek, Dipl.- Chem.,**
***Deichmannhaus am Hauptbahnhof, D-5000 Köln 1***
**(DE)**

LIBER, STOCKHOLM 1986

**Beschreibung**

Die Erfindung bezieht sich auf eine Ventilvorrichtung für den an beiden Enden offenen Kanal in dem Gehäuse eines medizinischen Instrumentes zur Herstellung eines Zuganges zu einem Blutgefäß oder Körperhohlraum mit einem als Ventilelement dienenden Schlauchabschnitt, der in dem Kanal wandschlüssig koaxial angeordnet ist und an den ein Klemmorgan angesetzt ist, das in dem Gehäuse zwischen Schließ- und Öffnungsstellungen für den Schlauchabschnitt quer zur Längsachse des Schlauchabschnittes verstellbar angeordnet ist.

Medizinische Instrumente zur Herstellung eines Zuganges zu einem Blutgefäß oder Körperhohlraum dienen beispielsweise zum Einführen von Kathetern und Sonden. Es handelt sich dabei um Einweginstrumente, die trotz einfachen und preiswerten Aufbaues hinsichtlich ihrer Ventilkonstruktion sicher sein müssen, damit der Kanal nach Herausnehmen der Punktionsnadel zuverlässig abgesperrt ist. Es ist bekannt (US-PS 40 00 739), bei derartigen Instrumenten geschlitzte oder gelochte gummielastische Ventilscheiben zu verwenden, die jedoch durch die eingelegte Punktionsnadel über den Hooke'schen Bereich überdehnt werden können, so daß es nach Herausziehen der Punktionsnadel zu Undichtigkeiten kommt. Ein weiterer Nachteil von Ventilscheiben besteht darin, daß sie das Hindurchschieben des Katheterschlauches erschweren und der Anwender besondere Sorgfalt darauf verwenden muß, daß sich das Instrument möglichst nicht bewegt, damit an der Einführungsstelle in den Körper des Patienten keine Reizungen oder Schädigungen auftreten.

Ferner ist die eingangs erwähnte Ventilvorrichtung bekannt (US-A-4 215 702), die jedoch ebenfalls mehrere Nachteile hat, und zwar 1. in Grundstellung, d.h. im unbenutzten Zustand, wird der Schlauchabschnitt von einem ihn umgebenden, als Klemmorgan dienenden Ring zusammengeklemmt, so daß er während des langen Zeitraumes von der Herstellung der Ventilvorrichtung bis zu ihrem Einsatz mechanisch beansprucht wird und an Flexibilität einbüßt mit der Folge, daß bei Verstellung des Klemmorgans zur Öffnung des Kanals des Schlauchabschnittes dieser sich selbsttätig nicht mehr so flexibel zurückstellen kann, daß der Kanal zum Durchlaß z.B. eines Katheters garantiert voll geöffnet ist; 2. das Klemmorgan wird von einem in bezug auf das die Ventilvorrichtung enthaltende Instrument weit nach außen ragenden klappbaren Hebel betätigt, der bei der Handhabung des Instrumentes im Wege ist und eine versehentliche Verstellung mit unerwünschter Beeinflussung des Schlauchabschnittes begünstigt; 3. der Hebel wird durch federelastische Eigenschaften des Materials, aus dem er und das Ventilgehäuse hergestellt sind, oder durch eine gesonderte Feder nach außen gedrückt, um das Klemmorgan in Absperrstellung zu halten. Zur Verstellung des Klemmorgans in Öffnungsstellung wird der Hebel gegen die Federkraft niedergedrückt und muß zur Offenhaltung des Schlauchabschnittkanals festgehalten werden.

Durch diese Mängel wird die bekannte Ventilvorrichtung sowohl hinsichtlich der vollen Öffnung als auch der vollständigen Absperrung des Schlauchabschnittkanals unzuverlässig und außerdem erschwert Punkt 3 die Handhabung. Würde man die bekannte Ventilvorrichtung z.B. für eine Schleuse zur Einführung eines Katheters oder einer Sonde in ein Blutgefäß benutzen, so würde der Anwender durch die Notwendigkeit des Niedergedrückthaltens des Hebels beim Vorschieben des Katheters behindert. Bereits leichtes Nachlassen des Druckes gegen den Hebel verschließt den Schlauchabschnittkanal und bremst den Kathetervorschub. Ferner wird es schwierig sein, das Instrument mit niedergedrücktgehaltenem Hebel so bewegungslos zu halten, daß die Einführungsstelle in den Körper des Patienten keine Reizungen oder Schädigungen erfährt.

Zum Öffnen und Schließen von Schlauchdurchlässen sind Schlauchklemmen bekannt (US-A-3 357 674), die als Schieber mit einem weiten und einem verengten Schlitzteil ausgebildet sind, deren Stellung in bezug auf den Schlauch die Funktion der Schlauchklemme bestimmt. Solche Schlauchklemmen sind separate Teile, die an Zu- oder Ableitungsschläuche medizinischer Geräte angesetzt werden, jedoch nicht als Klemmorgan für ein schlauchartiges Ventilelement in dem Gehäuse einer Ventilvorrichtung vervendbar sind.

Der Erfindung liegt die Aufgabe zugrunde, eine einfache Ventilvorrichtung der erwähnten Art so zu verbessern, daß sie zuverlässig ist und sich bequem handhaben läßt, so daß mit ihr ausgerüstete medizinische Instrumente für den Patienten sicherer werden.

Diese Aufgabe wird erfindungsgemäß dadurch gelöst, daß das Klemmorgan aus einem geraden Schieber besteht, der mindestens eine an den spannungsfrei in dem Kanal untergebrachten Schlauchabschnitt angreifende Keilprofilierung aufweist und länger ist als der Außendurchmesser des Gehäuses, und daß der Schieber in einem an beiden Enden offenen Querschlitz des Gehäuses gelagert und zwischen einer Klemmstellung und einer Freigabestellung für den Schlauchabschnitt hin- und herverschiebbar ist.

Eine solche Ventilvorrichtung zeichnet sich durch zuverlässige Absperrwirkung aus, die sich dadurch ergibt, daß das Ventilelement, d.h. der Schlauchabschnitt seine ursprüngliche Flexibilität uneingeschränkt beibehält, weil er ausschließlich in Schließstellung des Klemmorganes mechanisch beansprucht wird und in Öffnungsstellung der Ventilvorrichtung spannungsfrei in dem Kanal liegt. Da das Ventilelement nur zur Absperrung aktiviert wird, bleibt es maximal rückstellfähig und die Ventilvorrichtung kann unter Beibehaltung ihrer zuverlässigen Abdichtwirkung bzw. Gewährleistung vollständiger Öffnung des Schlauchabschnittquerschnitts beliebig oft geöffnet und geschlossen werden, wodurch sie für vielfältige Arten medizinischer Instrumente zur Herstellung eines Zuganges zu einem Blutgefäß oder Körperhohlraum geeignet ist. Die Handhabung ist einfach, weil der Schieber über das Gehäuse vorsteht und von dem Daumen in die eine Richtung und von dem Zeigefinger der gleichen Hand in die andere Richtung geschoben werden kann. Die beiden möglichen Stellungen des Schiebers bestimmen jeweils den geöffneten oder geschlossenen Zustand des Schlauchabschnittes, ohne daß der Schieber zur Fixierung des jeweiligen Zustandes festgehalten werden muß. Der Anwender des mit der Ventilvorrichtung ausgestatteten Instrumentes hat beide Hände frei, um z.B. einen Katheter durch den Kanal des Schlauchabschnitts in einen Körperhohlraum einzuführen. Außerdem ist vorteilhaft, daß die Ventilvorrichtung sich sehr preiswert herstellen läßt, so daß sie auch in dieser Hinsicht für Einweginstrumente gut geeignet ist.

Die dem Schlauchabschnitt zugewandte Keilprofilierung und die Abmessungen von Klemmorgan und Schlauchabschnitt bzw. Kanal sind so aufeinander abgestimmt, daß in Auf-Position die Erweiterung der Keilprofilierung die Ursprungsform des Schlauchabschnittes nicht beeinträchtigt, während in Zu-Position die Verengung der Keilprofilierung eine Zusammenpressung des Schlauchabschnittes bis zu seiner vollständigen Absperrung verursacht.

Zweckmäßigerweise ist die Keilprofilierung als Schlüssellochöffnung ausgebildet, und es ist der Querschnitt der kreisförmigen Zone der Schlüssellochöffnung dem Außendurchmesser des Schlauchabschnittes angepaßt. Die Schlüssellochöffnung kann geschlossen oder an ihrer Schmalseite offen ausgebildet sein. Im letzteren Falle erhält der Schieber Gabelform. Hierdurch wird die Montage der Ventilvorrichtung erleichtert, weil der Schlauchabschnitt nicht durch die kreisförmige Zone der Schlüssellochöffnung des in den Gehäuseschlitz eingeschobenen Schiebers hindurchgeführt werden muß, sondern zunächst der Schlauchabschnitt in den Kanal eingesetzt und erst dann der gabelförmige Schieber von der Seite her durch den Querschlitz über den Schlauchabschnitt geschoben wird.

Das Gehäuse kann als Katheteransatz eines Kurzkatheters zur Einführung von Kathetern und Sonden ausgebildet sein.

Bei der Montage werden der Schieber und der Schlauchabschnitt in das Gehäuse spannungsfrei eingelegt und anschließend wird die Punktionsnadel eingeführt. Hat der Anwender die Punktion durchgeführt, kann nach Herausnahme der Punktionsnadel durch Verstellung des Schiebers zur Zusammenpressung des Schlauchabschnittes der Kanal verschlossen werden. Durch den zusammengequetschten Schlauchabschnitt ist gewährleistet, daß kein Blut austritt. Wenn die Spitze des einzuführenden Katheters oder der Sonde in den Kanal eingesetzt ist, wird durch Verstellung des Schiebers nach der anderen Seite der Kanal geöffnet und es kann der Katheter in den Patienten eingeführt werden. Es ergibt sich eine zuverlässige Katheterschleuse einfachen Aufbaus.

Für einen weiteren Anwendungszweck der Ventilvorrichtung ist das Gehäuse als Anschlußstück für die Luftleitung eines Ballonkatheters ausgebildet. Dabei kann der Kanal in dem Gehäuse schräg in einen zweiten Kanal münden, an den der Ballonkatheter angeschlossen ist. In diesem Falle sperrt die Ventilvorrichtung die Luftleitung des Ballonkatheters, der ein Kardiologiekatheter sein kann, ab, nachdem der Ballon beispielsweise mit Hilfe einer geritzeaufgeblasen worden ist, die an das äußere Ende des Kanals in dem Gehäuse angesetzt wurde. Die luftdichte Absperrung des Kanals bei entsprechender Stellung des Schiebers verhindert ein Entweichen von Luft aus dem Ballon und dieser erfüllt seine Fixierungsfunktion in dem betreffenden Körperorgan über den gewünschten Zeitraum zuverlässig. Zur Entlüftung des Ballons wird der Schieber nach der entgegengesetzten Seite verstellt, damit der Schlauchabschnitt sich weitet und der Kanal geöffnet ist.

In der Zeichnung sind Ausführungsbeispiele der Erfindung schematisch dargestellt. Es zeigt:

Fig. 1 eine Katheterschleuse mit teilweise langsgeschnittenem Katheteransatz eines Kurzkatheters,

Fig. 2 einen Schnitt längs der Linie II-II in Figur 1 mit geöffneter Ventilvorrichtung,

Fig. 3 einen Schnitt entsprechend Figur 2 mit geschlossener Ventilvorrichtung,

Fig. 4 einen Längsschnitt des Einführungsendes der Katheterschleuse,

Fig. 5 einen Längsschnitt eines medizinischen Instrumentes mit einer Ventilvorrichtung für die Luftleitung eines Ballonkatheters,

Fig. 6 eine Ansicht desextrakorporalen Endes des Instrumentes mit geschlossener Ventilvorrichtung, und

Fig. 7 einen Schnitt längs der Linie VII-VII in Figur 5 mit geöffneter Ventilvorrichtung.

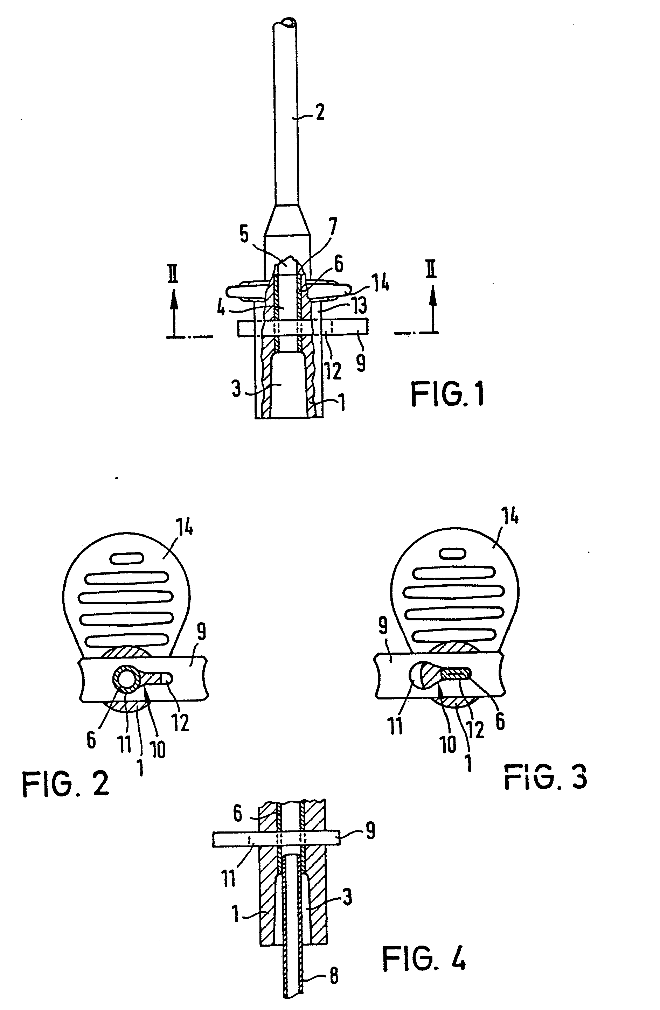

Gemäß Figur 1 besteht eine Katheterschleuse aus einem als Katheteransatz für einen Kurzkatheter 2 ausgebildeten Gehäuse 1, dessen Kanal 3, 4, 5 konzentrisch aufeinanderfolgende Teile aufweist. Der erste Teil 3 ist ein Innenkonus zur Aufnahme des Kegels eines Punktionsnadelansatzes, der zweite Teil 4 ist zylindrisch und enthält einenwandschlüssig, jedoch spannungsfrei eingesetzten Schlauchabschnitt 6 und der dritte Teil 5 hat einen etwas kleineren Durchmesser als der zweite Teil 4 und stellt den Anschluß zu dem Kurzkatheter 2 her. Der Schaduchabschnitt 6 stößt gegen die zwischen den Kanalteilen 4 und 5 gebildete Schulter 7, wobei die Wandstärke des Schlauchabschnittes 6 im wesentlichen der Breite der Schulter 7 entspricht, so daß zwischen dem Innenlumen des Schlauchabschnittes 6 und dem Teil 5 keine Stufe vorhanden ist, die den Durchgang eines Katheters 8 (Fig. 4) behindern könnte.

Der Schlauchabschnitt 6 gehört zu einer

Ventilvorrichtung, die mittels eines Klemmorganes betätigt wird. Figuren 2 und 3 zeigen ein Klemmorgan, das als plattenartiger gerader Schieber 9 gestaltet ist, der eine in Schieberlängsrichtung liegende Schlüssellochöffnung 10 aufweist. Die kreisförmige Zone 11 der Schlüssellochöffnung 10 ist so bemessen, daß sie den Schlauchabschnitt 6 verformungsfrei aufnimmt, so daß in Öffnungsstellung des Schiebers 9 (Figur 2) der Gesamtquerschnitt des Schlauchabschnittes 6 zur Hindurchführung eines langgestreckten Gegenstandes zur Verfügung steht. Die Verengung 12 der Schlüssellochöffnung 10 quetscht den Schlauchabschnitt 6 in Schließstellung des Schiebers 9 (Figur 3) derart zusammen, daß die inneren Wandflächen fest gegeneinander gepreßt sind und der Kanal 3,4,5 dicht abgesperrt ist, so daß kein Blut austritt. Der Schieber 9 ist in einem an beiden Enden offenen Querschlitz 13 in dem Gehäuse 1 axial verschiebbar gelagert, wobei der Querschlitz 13 den Schieber 9 im wesentlichen spielfrei führt.

Zur Einführung eines Katheters 8 wird nach der Punktion und Herausziehen der Punktionsnadel aus der Anordnung der Schieber 9 aus der öffnungsstellung nach Figur 2 in die Schließstellung nach Figur 3 geschoben, in der kein Blut austreten kann. Sodann wird bei geschlossener Ventilvorrichtung die Spitze des Katheters 8 durch den Teil 3 des Kanales in dem Gehäuse 1 ein Stück in den Schlauchabschnitt 6 hineingeschoben und durch Verschiebung des Schiebers 9 in Öffnungsstellung der Durchgang durch den Schlauchteil 6 freigegeben, so daß der Katheter 8 durch die Katheterschleuse in ein Blutgefäß oder einen Körperhohlraum eingebracht werden kann. Zum Festhalten der Katheterschleuse während der Punktion und während des Einführens des Katheters dient eine mit dem Gehäuse 1 verbundene Griffplatte 14.

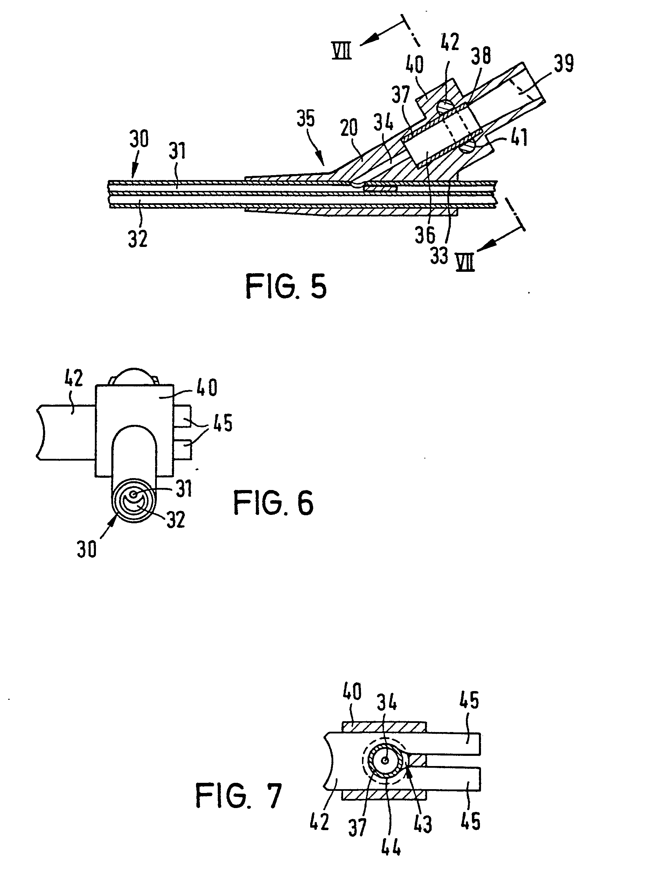

Bei dem Beispiel der Figuren 5 bis 7 wird eine abgewandelte Ausführungsform der Ventilvorrichtung für ein mit einem Ballonkatheter verbundenes medizinisches Instrument benutzt. In diesem Falle ist das Gehäuse 20 als Anschlußstück für die Luftleitung 31 eines Ballonkatheters 30 ausgebildet, dessen Flüssigkeitsleitung 32 durch einen Kanal 33 hindurchgeführt ist. Der Kanal 33 und der Kanal 34 in dem Anschlußstück 20 verlaufen Y-förmig in einem Y-Stück 35 aus Kunststoff. An den Kanal 34 schließt sich eine konzentrische zylindrische Erweiterung 36 an, in der ein Schlauchabschnitt 37 wandschlüssig untergebracht ist. Eine Schulter 38 am Übergang der Kanalerweiterung 36 zu einem Innenkonus 39 verhindert ein Herausrutschen des Schlauchabschnittes 37 aus dem Kanal. In einer eckigen Verbreiterung 40 des Gehäuses 20 ist ein Querschlitz 41 ausgebildet, in dem ein Schieber 42 axial hin- und herbeweglich gelagert ist.

Der Schieber 42 hat Gabelform, weil die Schmalseite der Schlüssellochöffnung 43 offen ist. Hierdurch wird die Montage der Ventilvorrichtung vereinfacht, weil zunächst der Schlauchabschnitt 37 in den Kanal 36 spannungsfrei eingesetzt und erst dann von der Seite her durch den Schlitz 41 der Schieber 42 über den Schlauchabschnitt 37 geschoben wird, bis die Kreisöffnung 44 den Schlauchabschnitt 37 aufnimmt.

Zum Aufblasen des Ballons des Ballonkatheters 30 wird der Kegel einer Spritze in den Innenkonus 39 des Gehäuses 20 hineingesteckt und die Spritze wird bei geöffneter Ventilvorrichtung (Figur 7) betätigt, bis etwa 1 ml Luft in den Ballon hineingedrückt worden ist. Anschließend wird der Schieber 42 aus der Stellung nach Figur 7 in die Stellung nach Figur 6 verschoben, so daß der Schlauchabschnitt 34 zwischen den Schenkeln 45 des Schiebers 42 zusammengequetscht wird. Der Kanal 34 ist dann so dicht abgesperrt, daß aus dem Ballon des Ballonkatheters 30 keine Luft entweichen kann. Nun kann durch die Flüssigkeitsleitung 32 des Ballonkatheters 30 Flüssigkeit in den Patienten eingeleitet werden.

## Patentansprüche

1. Ventilvorrichtung für den an beiden Enden offenen Kanal (4;36) in dem Gehäuse (1.20) eines medizinischen Instrumentes zur Herstellung eines Zuganges zu einem Blutgefäß oder Körperhohlraum mit einem als Ventilelement dienenden Schlauchabschnitt (6;37), der in dem Kanal (4;36) wandschlüssig koaxial angeordnet ist und an den ein Klemmorgan angesetzt ist, das in dem Gehäuse (1;20) zwischen Schließ- und Öffnungsstellungen für den Schlauchabschnitt (6;37) quer zur Längsachse des Schlauchabschnittes (6;37) verstellbar angeordnet ist, dadurch gekennzeichnet, daß das Klemmorgan aus einem geraden Schieber (9;42) besteht, der mindestens eine an den spannungsfrei in dem Kanal (4;36) untergebrachten Schlauchabschnitt (6;37) angreifende Keilprofilierung aufweist und länger ist als der Außendurchmesser des Gehäuses (1;20), und daß der schieber (9;42) in einem an beiden Enden offenen Querschlitz (13;41) des Gehäuses (1;20) gelagert und zwischen einer Klemmstellung und einer Freigabestellung für den Schlauchabschnitt (6;37) hin- und herverschiebbar ist.

2. Ventilvorrichtung nach Anspruch 1, dadurch gekennzeichnet, daß die Keilprofilierung als Schlüssellochöffnung (10;43) ausgebildet ist und daß der Querschnitt der kreisförmigen Zone der Schlüssellochöffnung (10;43) dem Außendurchmesser des Schlauchabschnittes (6;37) angepaßt ist.

3. Ventilvorrichtung nach Anspruch 2, dadurch gekennzeichnet, daß die Schlüssellochöffnung (10) geschlossen ausgebildet ist.

4. Ventilvorrichtung nach Anspruch 2, dadurch gekennzeichnet, daß die Schlüssellochöffnung (43) an ihrer Schmalseite offen ist und der Schieber (42) Gabelform hat.

5. Ventilvorrichtung nach einem der Ansprüche 1 bis 4,

<u>dadurch gekennzeichnet</u>, daß das Gehäuse (1) als Katheteransatz eines Kurzkatheters (2) zur Einführung von Kathetern (8) und Sonden ausgebildet ist.

6. Ventilvorrichtung nach einem der Ansprüche 1 bis 4,

<u>dadurch gekennzeichnet</u>, daß das Gehäuse (20) als Anschlußstück für die Luftleitung (31) eines Ballonkatheters (30) ausgebildet ist.

7. Ventilvorrichtung nach Anspruch 6,

<u>dadurch gekennzeichnet</u>, daß der Kanal (34,36) in dem Gehäuse (20) schräg in einen zweiten Kanal (33) mündet, an den der Ballonkatheter (30) angeschlossen ist.

## Claims

1. Valve device for the channel (4;36) open at both ends in the housing (1;20) of a medical instrument for establishing an access to a blood vessel or body cavity comprising a tube portion (6;37) which serves as a valve element and which, in contact with the wall, is coaxially arranged in the channel (4;36), a clamping member being applied to said tube portion and being adjustably provided in the housing (1;20) between closing and opening positions for the tube portion (6;37) in transverse direction to the longitudinal axis of the tube portion (6;37), characterized in that the clamping member is a straight slide (9;42) which contains at least one wedge profile engaging the tube portion (6;37) accomodated free of tension in the channel (4;36) and which is longer than the external diameter of the housing (1;20), and that the slide (9;42) being positioned in a double openended transverse slot (13;41) of the housing (1;20) is displaceable to and fro between a clamping position and a release position for the tube portion (6;37).

2. Valve device according to claim 1, characterized in that the wedge profile is of a keyhole opening design (10;43) and that the cross section of the circular zone of the keyhole opening (10;43) is adapted to the external diameter of the tube portion (6;37).

3. Valve device according to claim 2, characterized in that the keyhole opening (10) is of a closed design.

4. Valve device according to claim 2, characterized in that the narrow side of the keyhole opening (43) is open and the slide (42) is fork-shaped.

5. Valve device according to one of claims 1 to 4, characterized in that the housing (1) is a catheter hub of a short catheter (2) for introducing catheters (8) and probes.

6. Valve device according to one of claims 1 to 4, characterized in that the housing (20) is a connecting piece for the air conduit (31) of a balloon catheter (30).

7. Valve device according to claim 6, characterized in that the channel (34,36) in the housing (20) ends obliquely in a second channel (33) to which the balloon catheter (30) is connected.

## Revendications

1. Dispositif de soupape pour le canal (4; 36), ouvert des deux côtés, situé dans le boîtier (1; 20) d'un instrument medical servant à établir un accès à un vaisseau sanguin ou à une cavité du corps et comportant un élément de tuyau (6; 37) servant d'élément de soupape qui est disposé coaxialement dans le canal (4; 36) contre la paroi de ce dernier et sur lequel est monté un organe de serrage qui est disposé, dans le boîtier (1; 20), de manière à être déplaçable transversalement par rapport à l'axe longitudinal de l'élément de tuyau (6; 37), entre une position fermée et une position ouverte pour cet élément de tuyau, caractérisé en ce que l'organe de serrage est constitué par un curseur rectiligne (9; 42) qui possède au moins une forme profilée en coin s'appliquant contre l'élément de tuyau (6; 37) logé sans contrainte dans le canal (4; 36), et est d'une longueur supérieure au diamètre extérieur du boîtier (1; 20) et que le curseur (9; 42) est logé dans une fente tranversale (13; 41) du boîtier (1, 20), qui est ouverte au niveau de ses deux extrémités, et peut être déplacé en va-et-vient entre une position de blocage et une position de libération pour l'élément de tuyau (6; 37).

2. Dispositif de soupape selon la revendication 1, caractérisé en ce que la forme profilée en coin est realisée sous la forme d'une ouverture (10; 43) en forme de trou de serrure et que la section transversale de la zone de forme circulaire de l'ouverture (10; 43) du trou de serrure est adaptée au diamètre exterieur de l'élément de tuyau (6; 37).

3. Dispositif de soupape selon la revendication 2, caractérisé en ce que l'ouverture (10) en forme de trou de serrure est fermée.

4. Dispositif de soupape selon la revendication 2, caractérisé en ce que l'ouverture (43) en forme de trou de serrure est ouverte sur son côté etroit et que le curseur (42) possede la forme d'une fourche.

5. Dispositif de soupape selon l'une des revendications 1 à 4, caractérisé par le fait que le boîtier (1) est réalisé sous la forme d'un embout d'un cathéter court (2) permettant l'insertion de cathéters (8) et de sondes.

6. Dispositif de soupape selon l'une des revendications 1 à 4, caractérisé en ce que le boîtier (20) est réalisé sous la forme d'un élément de raccordement pour la canalisation d'air (31) d'un cathéter à ballon (30).

7. Dispositif de soupape selon la revendication 6, caractérisé en ce que le canal (34, 36) débouche obliquement, dans le boîtier (20), dans un second canal (33) auquel le cathéter à ballon (30) est raccordé.

**FIG.1**

**FIG. 2**

**FIG. 3**

**FIG. 4**

FIG. 5

FIG. 6

FIG. 7